# DEMANDE DE BREVET EUROPEEN

(11) **EP 2 730 647 A1**
(43) Date de publication de la demande: **14.05.2014**
(21) Numéro de dépôt: 13191962.3
(22) Date de dépôt: 07.11.2013
(51) Int. Cl.: C12N 1/12, A01G 33/00, A23K 1/00

(54) **Utilisation de sucre dans une culture de microalgues pour diminuer leur auto-floculation**

(30) Priorité: 09.11.2012 FR 1260680
(71) Demandeur: INVIVO NSA, 56250 Saint Nolff (FR)
(72) Inventeur: Devresse, Bernard, 2440 Geel (BE); Boisot, Pascal, 56660 Saint-Jean Brevelay (FR); Guyonvarch, Alain, 56000 Vannes (FR)
(74) Mandataire: Regimbeau

(57) **Abrégé**

L'invention concerne l'utilisation de sucre dans une culture de microalgues pour diminuer leur auto-floculation.

## Description

Les algues sont à la base de la chaîne aquatique alimentaire. Les micro-algues qui composent le phytoplancton jouent donc un rôle vital dans l'élevage des animaux aquatiques comme les mollusques, les crevettes et les poissons et présentent un intérêt stratégique en aquaculture. Au moment où la récolte et la pêche des populations sauvages ont atteint un stade critique, la contribution de l'aquaculture à la nutrition humaine est en constante progression.

Cet élément essentiel de la nutrition des mollusques, des crevettes et des poissons en aquaculture que sont les micro-algues fraîches n'a à ce jour pas trouvé d'équivalent. Les fermes aquacoles ont donc un besoin en algues fraîches qu'elles élèvent ou se fournissent commercialement.

Les microalgues fraiches constituent un risque sanitaire important pour les fermes aquacoles , qu'il leur faut maitriser soit par des livraisons quotidiennes des cultures fraiches commerciales, soit par une production sur le site même de la ferme, qui nécessite un investissement spécifique et conséquent et engendre des coûts d'exploitation importants.

Ce qui explique pourquoi les fermes aquacoles essaient de trouver des solutions alternatives à l'utilisation des microalgues fraiches.

Une solution pourrait consister à maitriser le risque sanitaire par l'utilisation de formes concentrées liquides.

Cependant , les formes concentrées, par exemple provenant d'une culture liquide atomisée ou lyophilisée, ne sont pas envisageables car les cellules s'agglomèrent lors du séchage et ne sont pas séparables lors de leur remise en suspension avant leur apport aux larves. Même une agitation très forte ne permet pas de les séparer. Une agitation très forte ne permet que de les tuer.

Ce phénomène est connu sous le nom d'autofloculation. Il existe également dans les cultures liquides.

Par exemple, interrompre l'apport en dioxyde de carbone d'une culture liquide peut causer une autofloculation c'est-à-dire une agglomération des cellules algales entre elles en « floculats ».

Dans les cultures extérieures, l'autofloculation semble associée au pH dû à la consommation de CO₂ par l'activité photosynthétique des algues. (Biotechnol Bioeng. 1984 Feb;26(2):142-7, Algal autoflocculation--verification and proposed mechanism, Sukenik A, Shelef G.).

La floculation chimique des micro-algues est également une méthode connue de séparation des algues de leur milieu en utilisant des floculants chimiques, comme le chlorure ferrique ou le chitosan issu de la chitine des carapaces des crustacés.

Des techniques de floculation chimiques permettent par exemple d'ôter les algues de l'eau et des eaux usées pour les purifier (Algal flocculation with synthetic organic polyelectrolytes, Tenney MW et al., Appl Microbiol. 1969 Dec;18(6):965-71). Les floculants utilisés sont des polyélectrolytes organiques synthétiques cationiques, anioniques, et non ioniques.

Cependant, toute floculation doit au contraire être évitée dans les cultures de micro-algues destinées à l'aquaculture puisque les cellules agglomérées n'ont pas la capacité de se désagglomérer lors de leur apport dans les bassins. Soit les cellules de micro-algues en sont détruites, soit les agglomérats sont trop importants en taille pour être ingérés par les larves de mollusques, de crevettes ou de poissons.

Le but de la présente invention est donc d'éviter l'autofloculation des micro-algues en culture liquide, lors de la préparation de concentrés de culture liquide et lors de la remise en suspension de concentrés de culture liquide.

De manière surprenante, les inventeurs ont observé que l'apport de sucre dans la culture liquide permet d'éviter l'autofloculation des micro-algues en culture liquide et la formation de floculats lors de la préparation de concentrés de cette culture liquide.

En particulier, ils ont observé que l'apport de 2% p/p de sucre à une culture de Chlorella concentrée jusqu'à environ 12% de matière sèche permet de diminuer voire d'éviter la formation de floculats après dilution de ce concentré.

Par conséquent, un premier objet de l'invention concerne un milieu de culture liquide pour la culture des micro-algues en suspension comprenant plus de 1,5 %, de préférence 2%, 3%, 5%, 10% en poids de sucre par rapport au volume dudit milieu liquide, de manière particulièrement préférée 2%.

Par « micro-algues », on entend au sens de la présente invention : les Chlorophycées, comme les *Chlorella, Closterium, Coelastrum, Dictyosphaerium, Scenedesmus, Selehastrum, Pediastrum, Staurastrum, Chlamydomonas, Tetraselmis,* les Diatomées, comme les *Phaeodactylum, Thalassiosira, Melosira, Asterionella, Cyclotella, Cymatopleura, Somphonema, Fragilaria, Stephanodiscus, Navicula, Skeletonema costatum,* les Euglénophycées, comme les *Phacus, Trachelomonas, Ceratium,* les Chrysophycées, comme les *Mallomonas, Dinobryon, Peridinium, Uroglena,* les Rhodophycées, comme les *Rhodella, Porphyridum, Cyanidium, Cyanidioschizon, Galdieria,* les Prymnesiophycées, comme les *Isochrysis, les Coscinodiscophycées,* comme les *Chaetoceros, Sheletonema, les Eustigmatophycies,* comme les *Nannochloropsis, les Thalassiosiracées,* comme les *Thalassiosira, les Paulovophycées,* comme les *Paulova.*

Par « sucre », on entend au sens de la présente invention les monosaccharides, disaccharides ou polysaccharides, avantageusement de moins de 20 résidus monosaccharidiques, notamment l'érythrose, le thréose, le ribose, l'arabinose, le xylose, le lyxose, l'allose, l'altrose, le glucose, le mannose, le gulose, l'idose, le galactose, le talose, l'érythrulose, le ribulose, le xylulose, le psicose, le fructose, le sorbose, le tagatose, le saccharose, le maltose et le lactose.

Les glucides contiennent du carbone, de l'hydrogène et de l'oxygène. Ces deux derniers atomes sont présents dans le même rapport 2 :1 que dans l'eau, c'est pourquoi les glucides sont parfois appelés hydrates de carbone.

Ce sont des molécules organiques caractérisées par la présence de chaînons carbonés porteurs de groupements hydroxyles, et de fonctions aldéhydes ou cétoniques.

En fonction de leur volume et de leur solubilité, les glucides sont classés en *monosaccharides* ou *oses* (1 sucre), en *disaccharides* ou *osides* (2 sucres), et en *polysaccharides* ou *polyosides* (nombreux sucres).

Les monosaccharides sont les unités de base de tous les autres glucides. En règle générale, plus la molécule de glucide est grosse, moins elle est soluble dans l'eau.

Les monosaccharides, ou *sucres simples,* sont formés d'une seule chaîne (linéaire ou cyclique) contenant 3 à 6 atomes de carbones. Leur formule générale est (CH₂O)n, n étant le nombre d'atomes de carbone. Les oses les plus abondants portent 5 ou 6 atomes de carbone, on les appelle des pentoses (n = 5, ex. le ribose et le désoxyribose) ou des hexoses (n = 6, ex. le glucose, le fructose et le galactose).

Un disaccharide est formé par la combinaison de 2 monosaccharides au cours d'une réaction de synthèse. Les deux molécules sont liées par une *liaison osidique* ou *liaison glycosidique* résultant de l'union de deux groupements hydroxyles avec perte d'une molécule d'eau. Les plus importants, de formule C₁₂H₂₂O₁₁, sont le saccharose, le maltose et le lactose.

Les polysaccharides sont les produits de polymérisation du glucose.

Le milieu de culture selon l'invention est avantageusement un milieu de culture connu de l'homme du métier pour la culture liquide des micro-algues en suspension supplémenté de plus de 1,5 % en poids de sucre par rapport au volume dudit milieu liquide, comme les milieux de Walnes, de Conway ou de Provasoli.

Composition du milieu de Walnes pour 1litre d'eau de mer:
- 2 mL de solution principale (solution principale pour 10 L : 680 g de nitrate de sodium, 200 g de di-hydrogénophosphate de sodium, 400 g d'acide éthylènediamine tétraacétique de sodium, 20 g d'acide borique, 800 mL de solution métallique, 40 mL de solution seawater, qsp d'eau déminéralisée)
- 0,2 mL de solution vitaminique (solution vitaminique pour 1L : 6 g de thiamine, 30 mL de milieu intermédiaire, qsp 1 L d'eau déminéralisée stérilisée)
   avec
- composition de la solution seawater pour 500 mL : 32,5 g de KBr, 6,5 g de chlorure de strontium, 0,25 g de chlorure d'aluminium, 0,1 g de chlorure de rubidium, 0,05 g de chlorure de lithium, 0,025 g de iodure de potassium, qsp 500 mL d'eau déminéralisée stérilisée
- composition du milieu intermédiaire pour 1 L : 1 g de biotine, 1 g de cyanocobalamine, qsp 1 L d'eau déminéralisée stérilisée.

Composition du milieu de Conway pour 1litre d'eau de mer:
- 1 mL de solution principale (solution principale pour 10 L: 1000 g de nitrate de sodium, 200 g de di-hydrogénophosphate de sodium, 450 g d'acide éthylènediamine tétraacétique de sodium, 336 g d'acide borique, 3,6 g de chlorure de manganèse, 13 g de chlorure ferrique, 10 mL de solution métallique, qsp d'eau déminéralisée)
- 0,1 mL de solution vitaminique (solution vitaminique pour 1 L : 2 g de thiamine, 0,1 g de cyanocobalamine, qsp 1 L d'eau déminéralisée stérilisée)
   avec
- composition de la solution métallique pour 1 L : 21 g de chlorure de zinc, 20 g de chlorure de cobalt hexahydraté, 20 g de sulfate de cuivre pentahydraté, 9 g d'ammonium heptamolybdate tétrahydraté, qsp 1 L d'eau déminéralisée stérilisée Composition du milieu de Provasoli pour 1 L d'eau de mer:
- 1 mL de solution I (solution I: 750 g de nitrate de sodium, qsp 10 L d'eau déminéralisée)
- 1 mL de solution II (solution II : 50 g de di-hydrogénophosphate de sodium, qsp 10 L d'eau déminéralisée)
- 1 mL de solution métallique (solution métallique pour 10 L : 31,5 g de chlorure de fer hexahydraté, 43,6 g d'acide éthylène diamine tétraacétique de sodium, 10 mL d'une solution de sulfate de cuivre pentahydraté à 9,8 g/L, 10 mL d'une solution de molybdate de sodium dihydraté à 6,3 g/L, 10 mL d'une solution de sulfate de zinc pentahydraté à 22 g/L, 10 mL d'une solution de chlorure de cobalt hexahydraté à 10 g/L, 10 mL d'une solution de chlorure de manganèse tétrahydraté à 180 g/L, qsp 10 L d' eau déminéralisée)
- 0, 5 mL de solution vitaminique (solution vitaminique pour 10 L : 2 g de thiamine, 10 mL d'une solution de cyanocobalamine à 1 g/L, 100 mL d'une solution de biotine à 0,1 g/L, qsp 10 L d'eau déminéralisée stérilisée).

Un autre objet de l'invention concerne l'utilisation dans une culture de microalgues de 1 à 20 % de sucre en poids par rapport au poids sec de microalgues pour diminuer leur auto-floculation.

On entend par « poids sec » le poids d'une culture de micro-algues présentant moins de 10% d'eau, de préférence moins de 6%, de manière particulièrement préférée 5%.

On entend par « auto-floculation » la capacité des algues à former des amas de cellules algales (ou agglomérats ou floculats) sans modification chimique de la culture.

Avantageusement, l'utilisation selon l'invention permet de diminuer la taille des floculats de micro-algues de plus de 250 µm de diamètre, de préférence de 120 à 250 µm, de manière particulièrement préférée de moins de 10 µm.

De manière particulièrement préférée, l'utilisation selon l'invention permet d'éviter tout type de floculats de microalgues, de plus de 20 µm de diamètre, de 10 à 20 µm, ou de moins de 10 µm.

Un autre objet de l'invention concerne un concentré de microalgues comprenant :
- 80 à 99 %, de préférence de 90 à 95%, en poids de microalgues par rapport au poids du concentré,
- 1 à 10 %, de préférence 3 à 5%, en poids d'eau par rapport au poids du concentré, 5 à 20%
- 1 à 20 %, de préférence de 2 à 10%, en poids de sucre par rapport au poids sec de microalgues.

Le concentré de l'invention peut être congelé.

Le concentré de l'invention est préparé à partir d'une culture de microalgues liquide en suspension à laquelle est ajouté 1 à 20 % en poids de sucre par rapport au poids sec de microalgues. Après cet ajout de sucre, la culture liquide est séchée pour atteindre moins de 10 % d'eau, de préférence moins de 7%, 6%, de manière particulièrement préférée moins de 5%. Ce séchage est réalisé par atomisation ou lyophilisation.

Après le séchage, le concentré de l'invention peut être congelé.

L'atomisation est en général réalisée à une température en dessous de 240°C, de préférence < 200°C et de manière optimale autour de 160°C.

Un autre objet de l'invention concerne un procédé de transport de microalgues, caractérisé en ce qu'une culture fraiche et liquide de microalgues est additionnée de 1 à 20 % en poids de sucre par rapport au poids sec de microalgues avant le transport sous forme liquide.

On entend par « culture fraîche » une culture de moins de 20 jours, de préférence moins de 10 jours, de manière particulièrement préférée de moins de 5 jours.

Les micro-algues sont transportées de leur lieu de production vers la ferme aquacole qui va les utiliser, ces deux lieux pouvant être séparés de plusieurs milliers de kilomètres. En effet, les micro-algues sont surtout produites en Asie, par exemple au Japon et les fermes aquacoles européenne peuvent se fournir en micro-algues auprès de ces producteurs asiatiques.

Le but du procédé de transport selon l'invention est d'éviter l'autofloculation résultant du stress du transport. Ainsi, les micro-algues peuvent être conservées plus longtemps et sont plus faciles à ingérer par les larves en aquaculture.

Un autre objet de l'invention concerne une culture fraîche, liquide et en suspension de microalgues comprenant 1 à 20 % en poids de sucre par rapport au poids sec de microalgues. Les micro-algues sont transportées dans des containers réfrigérés à 4°C par avion ou congelées dans les containers frigorifiques <18°C par bateau.

Un autre objet de l'invention concerne l'utilisation d'un concentré selon l'invention ou d'une culture fraîche, liquide et en suspension de microalgues selon l'invention pour nourrir les mollusques, les poissons, les larves de poissons, les crevettes ou les larves de crevettes en aquaculture.

Les mollusques susceptibles d'être élevés en aquaculture sont les huîtres, les moules, les palourdes, les pétoncles, les noix de saint Jacques, les coques, les bigorneaux.

Les poissons susceptibles d'être élevés en aquaculture sont les cyprinidés comme la carpe argentée, la carpe herbivore et la carpe marbrée, les salmonidés comme la truite arc-en-ciel et le saumon atlantique saumons du Pacifique, les tilapias, les poissons-chats ou siluriformes, comme le catfish, les clarias et les pangasius, les diadromes comme le milkfish et l'anguille, la sériole, la dorade japonaise, le bar, la dorade, le turbot, le barramundi (bar asiatique).

Les larves de poissons susceptibles d'être élevées en aquaculture sont les larves cyprinidés comme la carpe argentée, la carpe herbivore et la carpe marbrée, les salmonidés comme la truite arc-en-ciel et le saumon atlantique saumons du Pacifique, les tilapias, les poissons-chats ou siluriformes, comme le catfish, les clarias et les pangasius, les diadromes comme le milkfish et l'anguille, la sériole, la dorade japonaise, le bar, la dorade, le turbot, le barramundi (bar asiatique).

Les exemples suivants illustrent l'invention sans en limiter la portée.

### Exemples :

### Test de diminution des floculats avec différents sucres sur des Chlorelles

### Matériel et méthode

Le tableau ci-après représente les sucres testés à 16,66 % de poids sec ou 2% p/p avec 12% de poids sec d'algues.

| | | %poids sec | % p/p (avec 12% de poids sec d'algues) | | | |
|---|---|---|---|---|---|---|
| **Chlorelles** | Contrôle | Sans sucre | | | | |
| | C5 Xylose | 16,66 | **2** | | | |
| | C6 Fructose | 16,66 | **2** | | | |
| | C6 Glucose | 16,66 | **2** | | | |
| | C6 Mannose | 16,66 | **2** | | | |
| | C6 Inuline | 16,66 | **2** | | | |
| | C12 Maltose | 16,66 | **2** | | | |
| | C12 Saccharose | 16,66 | **2** | | | |
| | C12 Lactose | 16,66 | **2** | | | |
| | C18 Raffinose | 16,66 | **2** | | | |
| | Mannane | 16,66 | **2** | | | |
| | Gomme guar | 16,66 | **2** | | | |

10g du mélange algues +sucre sont mélangés dans 200 ml d'eau.

Le nombre de floculats par mL est compté au compteur cellulaire Thomas.

Les floculats sont classés en catégories 1 (inférieur à 20µm), 2 (entre 20 et 50 µm) et 3 (supérieur à 50 µm).

Le nombre de cellules est également compté par ml et après sédimentation (12-24h).

Les cultures sont diluées 2 fois et comptées.

### Résultats

| SUCRE | CONTRÔLE | C5 Xylose | C6 Fructose | C6 Glucose | C6 Mannose | C6 Inulin | C12 Maltose | C12 Saccharose | C12 Lactose | C18 Raffinose | Mannane | Gomme guar |
|---|---|---|---|---|---|---|---|---|---|---|---|---|
| agglomérats par ml | 7,60E+0 6 | 4,80E +06 | 2,40E+ 06 | 0,00E+ 00 | 0,00E+ 00 | 0,00E +00 | 0,00E+ 00 | 2,40E+06 | 0,00E+ 00 | 0,00E+00 | 0,00E +00 | 0,00E+0 0 |
| nr Aggl Cat 1 (<20µm) | 0,13 | 0,06 | 0,06 | 0 | 0 | | 0 | 0,06 | 0 | 0 | | 0 |
| nr Aggl Cat 2 (20-50µm) | 0,06 | 0,06 | 0 | 0 | 0 | | 0 | 0 | 0 | 0 | | 0 |
| nr Aggl Cath 3 (>50µm) | 0 | 0 | 0 | 0 | 0 | | 0 | 0 | 0 | 0 | | 0 |
| Cellules d'algues | 31,7 | 50 | 38,3 | 36,7 | 36,3 | | 45,3 | 33 | 35 | 34,7 | | 27,3 |
| Algues/ml | 1,27E+09 | 2,00E+09 | 1,53E+09 | 1,47E+09 | 1,45E+09 | 0,00E+00 | 1,81E+09 | 1,32E+09 | 1,40E+ 09 | 1,39E+09 | 0,00E+00 | 1,37E+09 |
| Algues/gramme de produit | 1,27E+11 | 2,00E+11 | 1,53E+11 | 1,47E+11 | 1,45E+ 11 | 0,00E+00 | 1,81E+11 | 1,32E+11 | 1,40E+11 | 1,39E+11 | 0,00E+00 | 1,37E+11 |
| Cellules d'algues comptées après 12h | | | | | | | | | | | | |
| Algues/ml | | | | | | | | | | | | |
| Cellules d'algues comptées après 24h | | | | | | | | | | | | |
| Algues/ml | | | | | | | | | | | | |

Test de dosage de la diminution des floculats avec du glucose sur des Chlorelles

### Matériel et méthode

| | | % p/p (avec 12% de poids sec d'algues) | | | | |
|---|---|---|---|---|---|---|
| Chlorelles (Fournisseur 1) | Contrôle | Sans sucre | | | | |
| | C6 Glucose | 1 | 5 | 10 | 20 | 30 |
| | % p/p | **0,12** | **0,6** | **1,2** | **2,4** | **3,6** |
| Chlorelles (Fournisseur 2) | C6 Glucose | 20 | **2,4** | | | |

10g/L d'algues séchées par atomisation sont mélangés dans 200 ml d'eau.
Le nombre de floculats par mL est compté au compteur cellulaire Thomas.

Les floculats sont classés en catégories 1 (inférieur à 20µm, 2 (entre 20 et 50 µm) et 3 (supérieur à 50 µm).

Le nombre de cellules est également compté par ml et après sédimentation (12-24h).

Les cultures sont diluées 2 fois et comptées.

### Résultats

| | | | | | |
|---|---|---|---|---|---|
| agglomérats par ml | 1,20E+06 | 2,60E+06 | 1,20E+06 | 0,00E+00 | 0,00E+00 |
| Aggl Cat 1 (<20µm) | 0 | 0,13 | 0,06 | 0 | 0 |
| Aggl Cat 2 (20-50µm) | 0 | 0 | 0 | 0 | 0 |
| Aggl Cat 3 (>50µm) | 0,06 | 0 | 0 | 0 | 0 |
| Cellules d'algues comptées | 48 | 35 | 37,7 | 31,3 | 39,7 |
| Algues/ml | 1,92E+09 | 1,40E+09 | 1,51 E+09 | 1,25E+09 | 1,59E+09 |
| Algues/gramme de produit | 1,92E+11 | 1,40E+11 | 1,51E+11 | 1,25E+11 | 1,59E+11 |
| Cellules d'algues comptées après 12h | | | | | |
| Algues/ml | | | | | |
| Cellules d'algues comptées après 24h | | | | | |
| Algues/ml | | | | | |

## Revendications

1. Utilisation dans une culture de microalgues de 1 à 20% de sucre par rapport au poids sec de microalgues pour diminuer leur auto-floculation.

2. Utilisation selon la revendication 1 pour éviter les floculats de moins de 20 µm de préférence moins de 10µm de diamètre.

3. Utilisation selon la revendication 1 ou 2, **caractérisée en ce que** lesdites microalgues sont des Chlorophycées ou des Diatomées.

4. Concentré de microalgues destiné à l'alimentation aquacole comprenant :
- 80 à 99 % en poids de microalgues par rapport au poids du concentré,
- 1 à 10 % en poids d'eau par rapport au poids du concentré,
- 1 à 20 % en poids de sucre par rapport au poids sec de microalgues.

5. Concentré de microalgues selon la revendication 4, congelé.

6. Procédé de préparation du concentré selon la revendication 4 ou 5, **caractérisé en ce que** l'on
- ajoute à une culture de microalgues 1 à 20 % en poids de sucre par rapport au poids sec de microalgues,
- sèche cette culture de microalgues jusqu'à moins de 10 % d'eau,
- éventuellement, on congèle.

7. Procédé de transport de microalgues, **caractérisé en ce qu'**une culture fraiche et liquide de microalgues est additionnée de 1 à 20 % en poids de sucre par rapport au poids sec de microalgues avant le transport sous forme liquide.

8. Culture fraîche, liquide et en suspension de microalgues comprenant 1 à 20 % en poids de sucre par rapport au poids sec de microalgues.

9. Utilisation d'un concentré selon la revendication 4 ou 5 ou d'une culture fraîche, liquide et en suspension de microalgues selon la revendication 8 pour nourrir les mollusques, les larves de poissons ou les larves de crevettes en aquaculture.

10. Milieu de culture liquide pour la culture des micro-algues en suspension comprenant plus de 1,5 % en poids de sucre par rapport au volume dudit milieu liquide **caractérisé en ce qu'**il s'agit du milieu de Walnes, de Conway ou de Provasoli supplémenté de plus de 1,5 % en poids de sucre par rapport au volume dudit milieu liquide.
